(19) Europäisches Patentamt / European Patent Office / Office européen des brevets

(11) **EP 3 216 490 A1**

(12) **EUROPEAN PATENT APPLICATION**

(43) Date of publication:
**13.09.2017 Bulletin 2017/37**

(51) Int Cl.:
*A61N 1/36* *(2006.01)*     *A61N 1/362* *(2006.01)*

(21) Application number: **17159839.4**

(22) Date of filing: **08.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.03.2016 US 201662306094 P**

(71) Applicant: **BIOTRONIK SE & Co. KG**
**12359 Berlin (DE)**

(72) Inventors:
- **Baru, Marcelo**
 **Tualatin, OR Oregon 97062 (US)**
- **Müssig, Dirk**
 **West Linn, OR Oregon 97068 (US)**
- **Stotts, Larry**
 **Tigard, OR Oregon 97224 (US)**

(74) Representative: **Keck, Hans-Georg**
**BIOTRONIK SE & Co. KG**
**Corporate Intellectual Properties**
**Woermannkehre 1**
**12359 Berlin (DE)**

(54) **SYSTEM AND METHOD FOR GENERATING PREMODULATED INTERFERENTIAL CURRENTS, PARTICULARLY FOR SPINAL CORD STIMULATION**

(57) The invention relates to a system and method for generating a premodulated interferential current, particularly for spinal cord stimulation, using a pulse generator having multiple electrodes, wherein a premodulated current is generated using at least one electrode, wherein said premodulated current comprises a train of biphasic pulses having a repetition frequency, wherein each biphasic pulse comprises a stimulating phase and a balancing phase, and wherein said premodulated current comprises an amplitude modulation envelope having an envelope beat frequency smaller than said repetition frequency of the biphasic pulses, wherein said modulation envelope is generated in the pulse generator.

FIG. 5

EP 3 216 490 A1

**Description**

The invention relates to a method and a system for generating premodulated currents, particularly for spinal cord stimulation (SCS).

A claimed advantage of stimulation via interferential currents (IFC) is increased stimulation depth reaching further into tissue. It is argued that in the intersection region, the reinforcement of the currents will result in greater total stimulus intensity so maximum stimulation is produced at depth rather than superficially as occurs with traditional bipolar stimulation. This is questionable as current spread reduces the intensity away from the electrodes. So even if the two currents are superimposed, the total intensity will most likely be less than immediately under the electrodes. Further, the IFC stimulus waveform seen by the targeted nerve fibers depend on the relative orientation with respect to the stimulating electrodes. Hence, the stimulus waveform created by conventional IFC has high degree of uncertainty.

Delivering two (or more) stimulus currents concurrently at different sites within a patient's body without crosstalk further presents implementation challenges for an implantable pulse generator (IPG) as both current generators are referenced to the battery of the IPG.

A possible solution is to have two or more entirely separate generators, powered by separate batteries. This however seems to be problematic when seeking to reduce size of an implantable pulse generator. US 8,977,363 B2 proposes another solution that has been traditionally used in external electrical stimulators which involves the use of transformer isolation to electrically float the generators. This however also has a negative bearing concerning the size of the system. Hence, stimulation via conventional IFC presents challenges for implementation in modern implantable IPGs that push the state-of-the-art in miniaturization.

Further, US 8,165,672 B2 discloses an SCS stimulation using a combination of high frequency (HF) signals and direct current (DC) signals.

Further, US2014/0257428 A1 discloses SCS stimulation patterns, wherein an envelope - HF stimulation - modulation signal is described.

Based on the above, the problem underlying the present invention is to provide a system and method of the afore-mentioned kind that allow to reduce the complications of implementing conventional IFC in an implantable pulse generator (IPG) as stated above.

This problem is solved by a method according to claim 1. Preferred embodiments are stated in the sub claims and are described below.

According to claim 1 a method for generating a premodulated interferential current, particularly for spinal cord stimulation (SCS), is disclosed, which uses a pulse generator (particularly an implantable pulse generator, i.e., a pulse generator that is configured to be implanted in a patient) having at least one or a plurality of electrodes, wherein a premodulated current is generated by the pulse generator and delivered using at least two electrodes, wherein said current comprises a train of biphasic pulses having a frequency $f_{train}$, wherein each biphasic pulse comprises a stimulating phase and a succeeding balancing phase, and wherein said premodulated current comprises an amplitude modulation envelope having an envelope beat frequency $f_{beat}$ being smaller than said frequency $f_{train}$ of the biphasic pulses, wherein said modulation envelope is generated in the pulse generator.

According to a preferred embodiment of the present method according to the invention, said biphasic pulses are rectangular pulses, wherein particularly each phase of the respective biphasic pulse is separated by an (e.g. programmable) interphase delay $T_D$.

Further, preferably, each balancing phase is followed by an open circuit phase where no current is applied via the electrodes. Preferably, during the open circuit phase the electrode potentials are indirectly monitored and particularly corrected if they exceed a safe voltage window allowing for uninterrupted therapy.

Further, according to a preferred embodiment of the present method according to the invention, the modulation envelope of said train ramps up in a linear fashion to a maximal amplitude and then ramps down in a linear fashion again.

Preferably, according to an embodiment, the phases of the individual pulses have the same pulse width (PW) delivered at a frequency preferably between 500 Hz and 20,000 Hz (that may ramp up and down in amplitude generating said modulation envelope, particularly with different programmable indexes). This permits combining the effects of highfrequency and low-frequency nerve fiber stimulation as desired.

It is known that nerves accommodate to a constant signal, tuning out the "electrical massage" effect caused by electrical stimulation to block pain, which is a major complication of traditional tonic-based spinal cord stimulation (SCS). This effect is known as habituation. To prevent this from occurring, in a preferred embodiment of the method according to the invention, the envelope beat frequency $f_{beat}$ is automatically varied by the pulse generator (or manually by the patient, e.g. via a remote control).

Here, preferably, in an embodiment, the envelope beat frequency $f_{beat}$ is changed by either automatically removing pulses from the train or automatically adding pulses to the train.

Preferably, in an embodiment, pulses are automatically added to the train or automatically removed from the train in a manner that translates into a gradual sweep of the envelope beat frequency $f_{beat}$, particularly in the form of a triangular pattern between a lower envelope beat frequency $f_{beatL}$ and a higher envelope beat frequency $f_{beatH}$.

Furthermore, according to a preferred embodiment, premodulated currents are delivered simultaneously using multiple electrodes to perform current steering, particularly for stimulation sweet spot identification. Current steering is understood as adjustment of the electrical stimulation field by changing the amplitudes of different current sources.

According to a preferred embodiment of the method according to the present invention, in order to prevent voltage runaway in the DC blocking capacitors placed in series (for protection) with the electrodes and electrode potential drifts (e.g. accumulated double layer voltages at the respective electrode-target double layer) into dangerous zones, allowing running electrical stimulation of the target continuously, the balancing phase for the pulses in the train is determined such that said capacitances (DC-blocking capacitor and double layer) charge in the same direction and opposite for stimulating and return electrodes. During the actual electrical stimulation of the target (e.g. SCS), indirect monitoring of the electrode potentials (e.g. accumulated double layer voltage), and corresponding correction (as required), is conducted in order to maintain the electrode potentials within safe windows of operation avoiding tissue damage or electrode corrosion.

According to a preferred embodiment of the method according the invention, for automatic charge balancing during a stimulation stage, in which a premodulated current is applied to a target, stimulating phases and balancing phases for the respective electrode are automatically programmed in a determination stage preceding said stimulation stage such that the difference between the circulating balancing current pulse minus the respective stimulation current pulse through the respective electrode satisfy:

- For at least one stimulating electrode, the difference equals an automatically determined positive value which may be different for different electrodes;
- For at least one returning electrode, the difference is positive and smaller or equal than the minimum among the difference values for the stimulating electrodes;
- For each electrode, both a DC blocking capacitor which couples the respective electrode to a current source, a current sink, or a voltage, and a double layer capacitance, wherein the double layer capacitance is formed by each electrode and the respective adjacent material, charge in the same direction and opposite for stimulating and returning electrodes, and wherein
- in said stimulation stage at least one of the electrodes is monitored, wherein, when a voltage accumulated at the double layer of the at least one monitored electrode crosses pre-defined thresholds, correction currents are generated and applied that reduce or cancel said accumulated voltages.

In this regards, according to a preferred embodiment of the method according the invention, for automatic charge balancing during a stimulation stage, in which at least one premodulated current is applied to a target, programmed stimulation currents $I_{Ni}$ and automatically-determined balancing currents $I_{Pi}$ (of said premodulated current) for the respective electrode "i" are programmed in a determination stage preceding said stimulation stage such that said premodulated current results and the difference (real $I_{Pi}$ - real $I_{Ni}$) (where real means actual value of the circulating current) between the real parts of the respective balancing phase $I_{Pi}$ and the respective stimulating phase $I_{Ni}$ through the respective electrode "i" satisfy:

- For stimulating electrodes, the difference equals an automatically determined positive value $I_{Diffi}$ (which may be different for different electrodes);
- For returning electrodes, the difference is positive and smaller or equal than $I_{MinDiff}$ where $I_{MinDiff}$ is selected equal to the smallest among the $I_{Diff}$ values for the stimulating electrodes. This programming ensures that at each electrode "i" both said DC blocking capacitor ($C_i$) connected in series with the respective electrode and the associated double layer capacitance ($C_{dli}$) formed by the respective electrode with adjacent target material charge in the same direction and opposite for stimulating and return electrodes, and wherein

  - in a stimulation stage succeeding said determination stage, a stimulation current pulse followed by a balancing current pulse is repeatedly applied via the at least one stimulating electrode and a return electrode to the target, wherein said pulses are programmed in beforehand in said determination stage, and wherein between a programmed balancing current pulse and the next programmed stimulation current pulse an open circuit phase is conducted where no current is imposed via the at least one stimulating electrode, and wherein

in said stimulation stage (particularly during the respective open circuit phase) at least one of the electrodes is indirectly monitored (i.e. without direct current connection), wherein, when a voltage ($\Delta V_{dli}$) accumulated at the double layer of the at least one monitored electrode crosses pre-defined thresholds, correction currents are automatically injected that reduce or cancel said accumulated voltages (including also all others that did not reach a threshold).

Preferably, in an embodiment, crossing of the thresholds by said accumulated double layer voltage is automatically detected during therapy delivery by comparing the voltage on the terminal of the DC-blocking capacitor opposite to the electrode under consideration against the difference between an internally-generated voltage reference and the estimated accumulated voltage at the DC blocking capacitor.

Preferably, in an embodiment, said current difference (i.e. the necessary charge imbalance) is determined for different patient postures and/or depending on the stimulation frequency.

Further, preferably, determining said current difference involves letting each stimulating electrode stimulate against a reference electrode in the determination stage, wherein particularly said reference electrode is formed by a casing of the pulse generator.

According to a further aspect of the present invention solving the above-stated problem, a system having the features of claim 9 is disclosed. Preferred embodiments of the system are stated in the corresponding sub claims and are stated below. All features described in conjunction with the method according to the invention may be used to further characterize the system according to the invention which is particularly configured to conduct the individual features / method steps of the method according to the invention. Vice versa, all features described in context with the system according to the present invention may be used to characterize individual steps of the method according to the invention.

According to claim 9, the system is preferably configured for spinal cord stimulation (SCS) and for generating a premodulated current (e.g. for said stimulation), wherein the system comprises a pulse generator (particularly an implantable pulse generator, see above) comprising at least one electrode or a plurality of electrodes, wherein the pulse generator is configured to generate a premodulated current using at least one electrode, wherein said premodulated current comprises a train of biphasic pulses having a frequency $f_{train}$, wherein each biphasic pulse comprises a stimulating phase and a succeeding balancing phase, and wherein said premodulated current comprises an amplitude modulation envelope having an envelope beat frequency $f_{beat}$ being smaller than said frequency $f_{train}$.

According to a preferred embodiment of the system according to the invention, said biphasic pulses are rectangular pulses, wherein particularly each phase of the respective biphasic pulse is separated by an (e.g. programmable) interphase delay (see also above).

Further, preferably, each balancing phase is followed by an open circuit phase where no current is applied via the electrodes. Preferably, during the open circuit phase the electrode potentials are indirectly monitored and particularly corrected if they exceed a safe voltage window (see below).

Further, according to a preferred embodiment of the system according to the invention, the pulse generator is configured to generate said premodulated current such that the modulation envelope of said train ramps up linearly to a maximal amplitude and then ramps down to a minimal amplitude.

Further, according to a preferred embodiment of the system according to the invention, the phases of the individual biphasic pulses comprise the same pulse width (PW), wherein the biphasic pulses are delivered with a frequency $f_{train}$ in the range from preferably 500 Hz to 20,000 Hz (see also above).

Further, according to a preferred embodiment of the system according to the invention, the pulse generator is configured to vary the envelope beat frequency $f_{beat}$ or may alternatively or in addition comprise a means configured for manually adjusting the beat frequency (e.g. by the patient, e.g. via a remote control).

Further, according to a preferred embodiment of the system according to the invention, the pulse generator is configured to automatically change the envelope beat frequency by either removing pulses from the train or by adding pulses to the train.

Further, according to a preferred embodiment of the system according to the invention, the pulse generator is configured to add pulses to the train or to remove pulses from the train in a manner that translates into a gradual sweep of the envelope beat frequency, particularly in the form of a triangular pattern between a lower envelope beat frequency $f_{beatL}$ and a higher envelope beat frequency $f_{beatH}$.

Further, according to a preferred embodiment of the system according to the invention, the pulse generator is configured to simultaneously deliver premodulated currents using multiple electrodes to perform current steering, particularly for stimulation sweet spot identification.

Furthermore, as already described above, the system is preferably also configured for conducting an automated charge balancing.

In particular, in a preferred embodiment of the present invention, the system is configured to automatically program stimulating phases and balancing phases for the respective electrode in a determination stage preceding said stimulation stage such that the difference between the circulating balancing current pulse minus the respective stimulation current pulse phase through the respective electrode satisfy:

- For at least one stimulating electrode, the difference equals an automatically determined positive value which may be different for different electrodes;
- For at least one returning electrode, the difference is positive and smaller or equal than the minimum among the difference values for the stimulating electrodes;
- For each electrode, both a DC blocking capacitor ($C_i$) which couples the respective electrode to a current source, a current sink or a voltage, and a double layer capacitance ($C_{dli}$), wherein the double layer capacitance ($C_{dli}$) is formed by each electrode and the respective adjacent material, charge in the same direction and opposite for stimulating and returning electrodes, and wherein

- in said stimulation stage at least one of the electrodes is monitored, wherein, when a voltage ($\Delta V_{dli}$) accumulated at the double layer of the at least one monitored electrode crosses pre-defined thresholds ($-\Delta V_{AddOCP}$, $\Delta V_{SubOCP}$), correction currents ($I_{CORRStim}$, $I_{CORRRet}$) are generated and applied that reduce or cancel said accumulated voltages ($\Delta V_{dli}$).

According thereto, according to a preferred embodiment of the system according the invention, for automatic charge balancing during a stimulation stage, in which at least one premodulated current is applied to a target, programmed stimulation currents $I_{Ni}$ and automatically-determined balancing currents $I_{Pi}$ (of said premodulated current) for the respective electrode "i" are programmed in a determination stage preceding said stimulation stage such that said premodulated current results and the difference (real $I_{Pi}$ - real $I_{Ni}$) (where real means actual value of the circulating current) between the real parts of the respective balancing phase $I_{Pi}$ and the respective stimulating phase $I_{Ni}$ through the respective electrode "i" satisfy:

- For stimulating electrodes, the difference equals an automatically determined positive value $I_{Diffi}$, (which may be different for different electrodes);
- For returning electrodes, the difference is positive and smaller or equal than $I_{MinDiff}$ where $I_{MinDiff}$ is selected equal to the smallest among the $I_{Diffi}$, values for the stimulating electrodes. This programming ensures that at each electrode "i" both said DC blocking capacitor ($C_i$) connected in series with the respective electrode and the associated double layer capacitance ($C_{dli}$) formed by the respective electrode with adjacent target material charge in the same direction and opposite for stimulating and return electrodes, and wherein

- in a stimulation stage succeeding said determination stage, a stimulation current pulse followed by a balancing current pulse is repeatedly applied via the at least one stimulating electrode and a return electrode to the target, wherein said pulses are programmed in beforehand in said determination stage, and wherein between a programmed balancing current pulse and the next programmed stimulation current pulse an open circuit phase is conducted where no current is imposed via the at least one stimulating electrode, and wherein
- in said stimulation stage (particularly during the respective open circuit phase) at least one of the electrodes is indirectly monitored (i.e. without direct current connection), wherein, when a voltage ($\Delta V_{dli}$) accumulated at the double layer of the at least one monitored electrode crosses pre-defined thresholds, correction currents are automatically injected that reduce or cancel said accumulated voltages (including also all others that did not reach a threshold).

Further, in an embodiment, the system is configured to automatically detect crossing of threshold by said accumulated double layer voltage by comparing the voltage on the terminal of the DC blocking capacitor opposite to the electrode under consideration against the difference between an internally-generated voltage reference and the estimated accumulated voltage at the DC blocking capacitor..

Further, in an embodiment, the system is configured to determine said current difference (i.e. the necessary charge imbalance) for different patient postures and/or depending on the stimulation frequency.

Further, in an embodiment, the system is configured to determine said current difference with help of letting each stimulating electrode stimulate against a reference electrode in a determination stage, wherein particularly said reference electrode is formed by a casing of the pulse generator.

DESCRIPTION OF THE DRAWINGS

Further features, embodiments and advantages of the present invention shall be described with reference to the Figures, wherein

Fig. 1          shows a system according to a preferred embodiment of the invention, which is particularly adapted for spinal cord stimulation (SCS) and may comprise an implantable pulse generator;

Fig. 2          shows a possible front-end of the pulse generator of Fig. 1;

Fig. 3          shows a premodulated current as generated with the system/method according to a preferred embodiment of the invention;

Fig. 4  shows a varying beat frequency generated with the system/method according to a preferred embodiment of the invention;

Fig. 5  shows a guarded cathode configuration of a system according to a preferred embodiment of the invention;

Fig. 6  shows the potential of a stimulating electrode when an active chargebalanced stimulation protocol is used in a high rate pulsing application;

Fig. 7  shows a schematic representation of the front-end of the implantable pulse generator of the system according to a preferred embodiment of the invention;

Fig. 8  shows an example of a stimulation phase and a balance phase of the pulse generator of Fig. 7;

Fig. 9  shows a circuit of the system according to the invention for determining the open circuit potential (OCP);

Fig. 10  shows the circuit of Fig. 9 to measure the accumulated voltage in cycled electrode "i" during the determination stage connected to the casing of the pulse generator;

Fig. 11  shows an N to 3 multiplexer (MUX) block for measuring voltages (a) and monitoring electrodes (b);

Fig. 12  shows a stimulation and balance phase following the determination stage in the example described;

Figs. 13 and 14  show comparators for indirectly monitoring the accumulated double layer voltages;

Fig. 15  show a correction phase for cancelling accumulated double layer voltages;

Fig. 16  show a compare phase preceding a correction phase; and

Fig. 17  shows a comparator for stopping the injection of a correction phase;

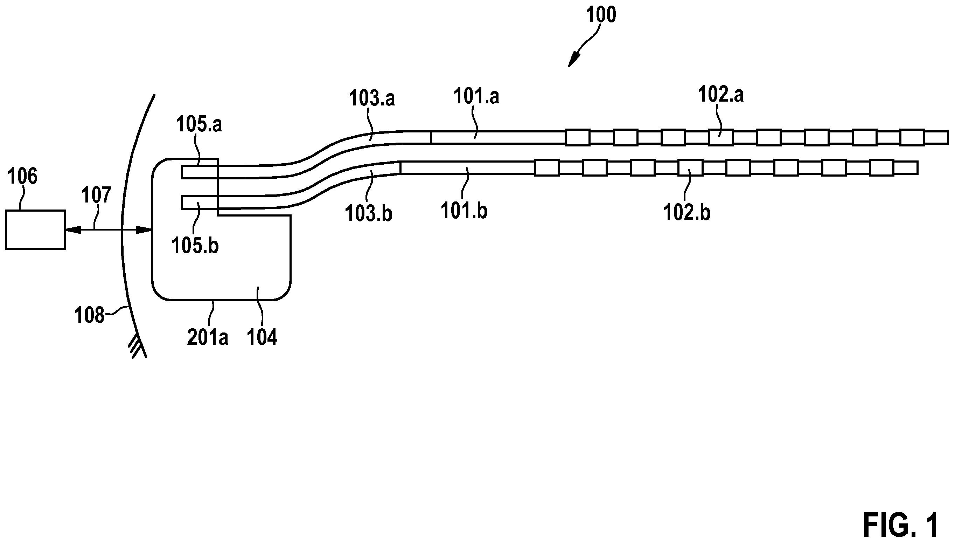

Figure 1 illustrates an example of an implantable system 100 for spinal cord stimulation (SCS). Such a system 100 includes first and second implantable percutaneous leads 101.a and 101.b that are configured to be implanted into a targeted location in the epidural space. Such leads 101 may be replaced by a paddle lead or other type of SCS leads.

The distal portion of the leads 101.a and 101.b incorporate a plurality of electrodes 102.a and 102.b respectively. Octal leads 101 (eight electrodes each) are shown in the example illustrated in Figure 1. Each electrode 102 is connected to an insulated wire (not shown) that run inside flexible insulated carriers 103.a and 103.b. These carriers 103 get tunneled during implantation to the vicinity of the implantable pulse generator (IPG) 104 that is typically implanted subcutaneously in the patient's lower abdominal or gluteal region. Carriers 103.a and 103.b terminate proximally in connectors 105.a and 105.b respectively that are then inserted into the IPG 104 header to allow conducting electrical charge to electrodes 102. The IPG 104 case is made of a material that approximates a pseudo reference electrode (e.g. fractal Ir or TiN) and with an effective area that makes its double-layer capacitance much larger than any of the electrodes 102.

The implantable pulse generator (IPG) 104 can particularly communicate with external devices 106 through suitable radio frequency (RF, e.g. MICS-band) or inductive links 107 that pass through the patient's skin 108. The external devices 106 may include a clinician programmer, a patient remote, or an external charger among others. Preferably, an external charger will send power transcutaneously through an inductive link 107 for battery recharge if the IPG 104 is powered by a secondary battery.

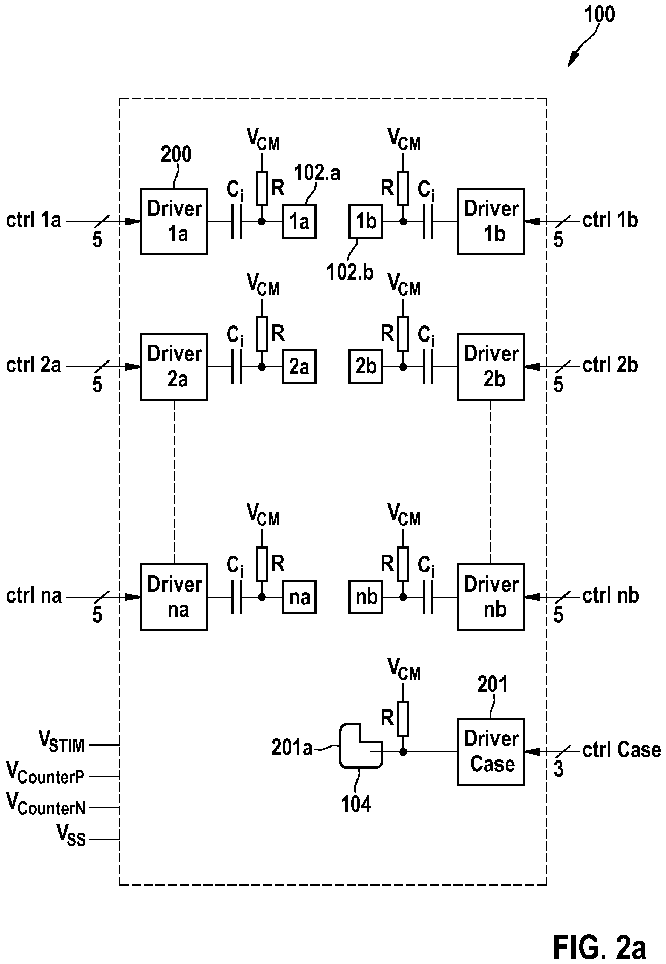

In a preferred embodiment of the system 100 according to the invention, the IPG 104 has the stimulation front-end shown in Figure 2a. Electrodes 102.a and 102.b are represented by elements $X_a$ and $X_b$ (X = 1..N) respectively. There are DC blocking capacitors $C_i$ in series with each electrode $X_a$, $X_b$ and these electrodes can be driven by circuitry in blocks 200. The IPG 104 casing 201a, on the other hand, can be driven by block 201 instead. Resistors R, connected to a common point $V_{CM}$, are charge bleeding-off resistors.

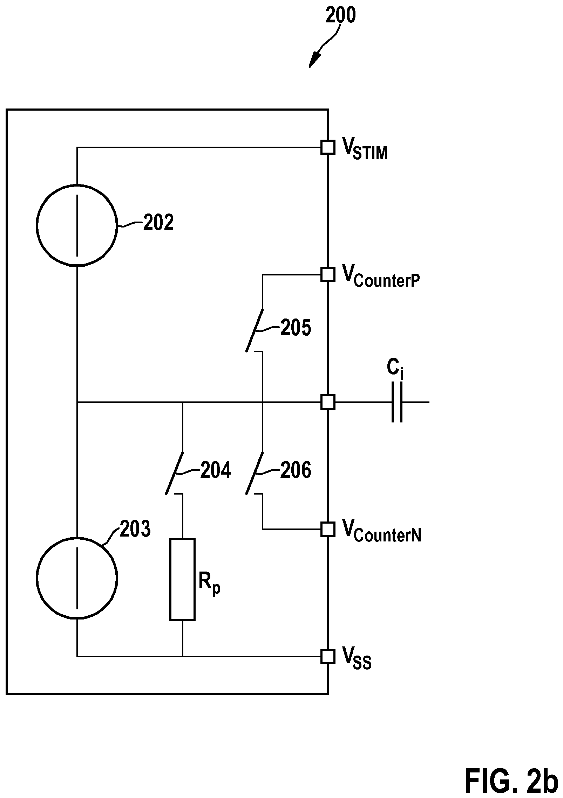

Each block 200 has five controllable elements as shown in Figure 2b where only one may be active at any time when the respective electrode is utilized for delivery of electrical (e.g. spinal cord) stimulation. Current 202 permits sourcing current through an electrode 102 from a programmable voltage $V_{STIM}$ (typically up to 16.0 V) whereas current 203 permits sinking current to $V_{SS}$ (system ground, typically the battery negative voltage) as desired. Having sourcing and sinking currents independently controllable at each electrode 102 permits delivering simultaneous multi-electrode SCS with active charge balancing, thus higher frequency, and applying current steering to enable targeted stimulation of specific nerve fiber populations. For low frequency applications, analog switch 204 and current limiting resistor $R_P$ permit passive charge balancing to minimize power consumption. Although the connection of $R_P$ is shown to $V_{SS}$, other intermediate common potentials may be utilized for passive charge balance.

For active charge balancing, analog switches 205 and 206 permit currents to circulate from voltage $V_{CounterP}$ or to voltage $V_{CounterN}$ respectively. Typically, $V_{CounterP}$ will be close to $V_{STIM}$ while $V_{CounterN}$ will be close to $V_{SS}$. In some cases, depending on the impedance and programmed stimulation current, $V_{CounterN}$ and $V_{CounterP}$ need to be offset up to 2.0 V from $V_{STIM}$ or Vss to prevent the circuitry in blocks 200 from exceeding $V_{STIM}$ or going below $V_{SS}$ which would trigger undesired parasitic conduction of solid-state elements in these blocks 200.

The IPG 104 case driver 201, on the other hand, only needs to include the analog switches 204..206 and the current limiting resistor $R_P$.

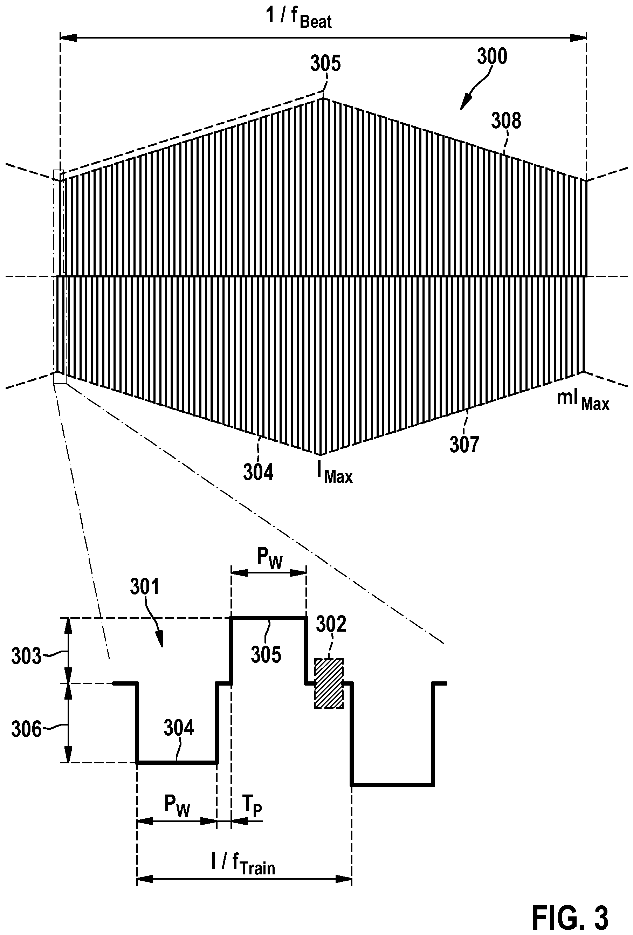

In a preferred embodiment of the system 100 according to the invention, two electrodes 102 from the same lead 101, e.g. 3a and 4a from lead 101.a, or an electrode 102 and the IPG 104 case, are used for stimulation using a premodulated current 300 generated inside the IPG 104 as shown in Figure 3. Such current can be preferably defined by the user via a clinician programmer by specifying the following parameters:

- frequency of train of biphasic pulses $f_{Train}$ (equivalent to sinusoidal carrier frequency in conventional interferential current IFC);
- maximum stimulation amplitude $I_{Max}$;
- amplitude modulation index m;
- and envelope beat frequency $f_{Beat}$.

The first parameter is preferably programmable in the 500 Hz - 20,000 Hz range, and at least up to 16,384 Hz. Each biphasic pulse 301 has identical duration of its phases with a programmable pulse width (PW) between 10 μs to 1,000 μs. The two phases are preferably separated by a programmable interphase delay $T_D$ in the range of 10 μs to 100 μs. For example, if $f_{Train}$ is programmed equal to 4,000 Hz (i.e. 250 μs period), PW and $T_D$ can be set to 110 μs and 10 μs respectively which leave up to 20 μs for open circuit phase 302. Such phase preferably 302 follows the end of the second phase of each biphasic pulse 301 and it is preferably used to indirectly monitor the electrode potentials and correct them as will be generally described below if they exceed a safe voltage window (typically ±100 mV).

As described above, in an embodiment, the first programmable parameter $f_{Train}$ dictates parameter PW which in turn limits parameter $I_{Max}$ as the charge injected per pulse shall not exceed the maximum allowable charge injection (typically in the order of 10 μC for SCS). Given this constraint, the range of programmable amplitudes $I_{Max}$ typically goes up to 25 mA. The modulation can be programmed on-off and when programmed on the amplitude modulate index m can be preferably programmed in eight discrete steps from 0.125 to 1.000. Figure 3 shows an index m equal to 0.500 (i.e. 50% modulation).

The number of pulses in each ramp up (down) is preferably in the range of 16 to 128. Together with parameter $f_{Train}$, they determine the possible programmable envelope beat frequencies $f_{Beat}$. In the example, with $1/f_{Train}$ set at 250 μs (i.e. 4,000 Hz), $1/f_{Beat}$ can have programmable values in the range of approximately 8 ms to 64 ms. Hence, it is possible with this example to deliver an envelope in the range of traditional tonic stimulation frequencies (typically 40 Hz, a 25 ms period).

According to a preferred embodiment, for a given stimulation ramp 304, the stimulation control logic in the IPG 104 automatically determines the balance phase amplitude 303 of each pulse 301 in the train 300 by applying a determination stage methodology as will be described below. In this case, the M determination pulses are "balanced as-programmed ramping pulses". Since Faradaic charge transfer is typically required to elicit a physiological response via electrical stimulation, this may result in the envelope of the balancing phases 305 having lower amplitude than that of the pulses in the stimulation ramp 304 as the preferred biphasic stimulation will be determined to be unbalanced with balancing phase amplitude 303 being less than the stimulating phase amplitude 306 for each pulse 301. During the actual delivery of electrical stimulation to the target in the stimulation stage, indirect monitoring of the electrode potentials and corresponding correction (as required) occurs during the open circuit phase 302 as will be described below.

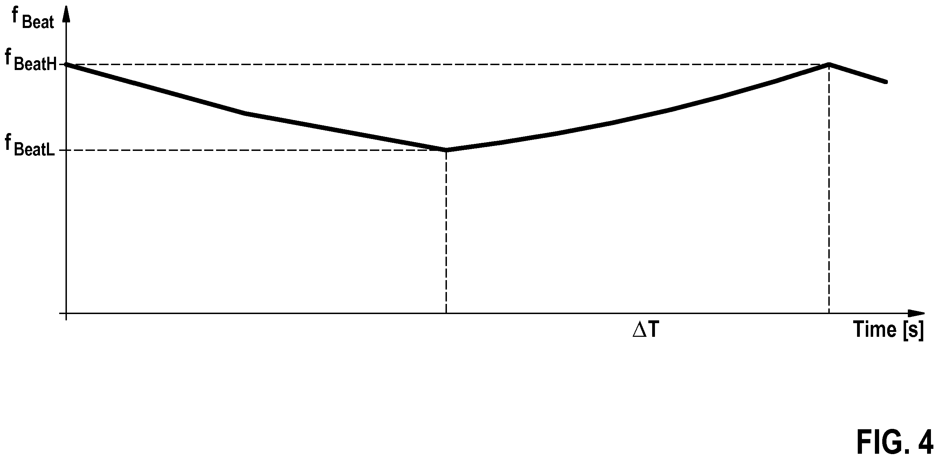

In another preferred embodiment, the envelope beat frequency $f_{Beat}$ is automatically swept in time by the control logic in the IPG 104, or manually swept by the patient using a remote control, between two limits $f_{BeatH}$ and $f_{BeatL}$ to prevent e.g. habituation to stimulation. Figure 4 shows the approximate triangular sweep pattern. Such sweep is achieved by adding or removing pulses 301 from the ramps 307, 308 on consecutive envelope periods and during a time ΔT. In the example being considered (i.e. $1/f_{Train}$ equal to 250 μs), is it possible for example to inject ramps each one consisting of 42 to 62 pulses 301 to generate $f_{Beat}$ frequencies that vary between a $f_{BeatH}$ and $f_{BeatL}$ equal to approximately 48 Hz and 32 Hz respectively. This provides a ±20% jitter around the traditional 40 Hz default tonic SCS frequency. The ΔT parameter may be programmable in the range of 1.0 s to 6.0 s. An alternative sweeping pattern (not shown) is a rectangular sweep where $f_{Beat}$ periodically changes discretely between $f_{BeatH}$ and $f_{BeatL}$ without going through intermediate frequencies.

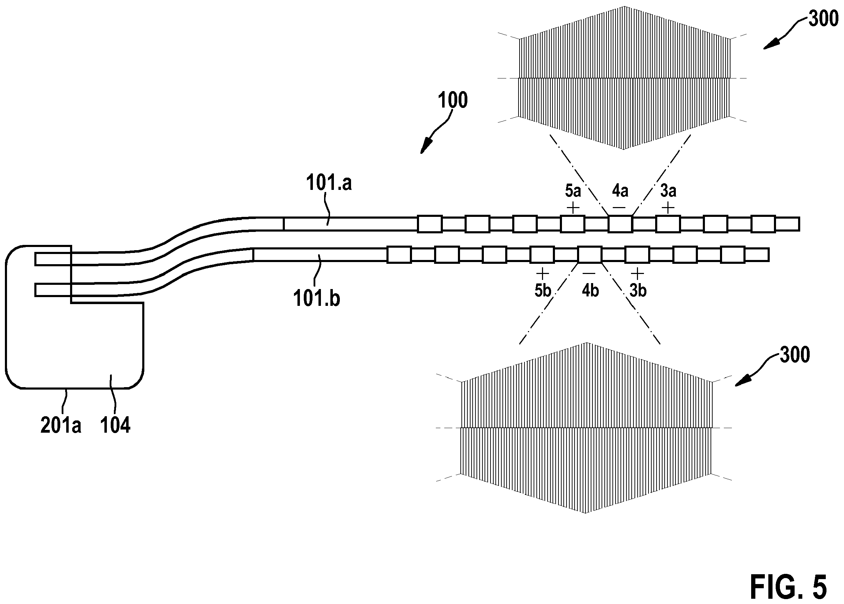

In another preferred embodiment, more than one premodulated current 300 is delivered simultaneously allowing for current steering stimulation. A preferred guarded cathode configuration is shown in Figure 5. Electrodes 3a, 3b and 5a, 5b of leads 101.a and 101.b operate as anodes whereas electrodes 4a, 4b of both leads 101.a, 101.b operate as cathodes for delivering electrical stimulation, i.e. said currents 300. Different weights to the stimulating currents (i.e. fractional currents) 300 can be programmed through electrodes 4a, 4b to permit targeting more central points of stimulation on the dorsal column.

Summarizing, the system 100 and methods of the present invention permit delivering simultaneous, multi-electrode stimulation with similar effects to conventional interferential currents (IFC) but particularly without modifying the architecture of the implantable pulse generator (IPG) 104 designed to deliver either low or high frequency pulsed stimulation.

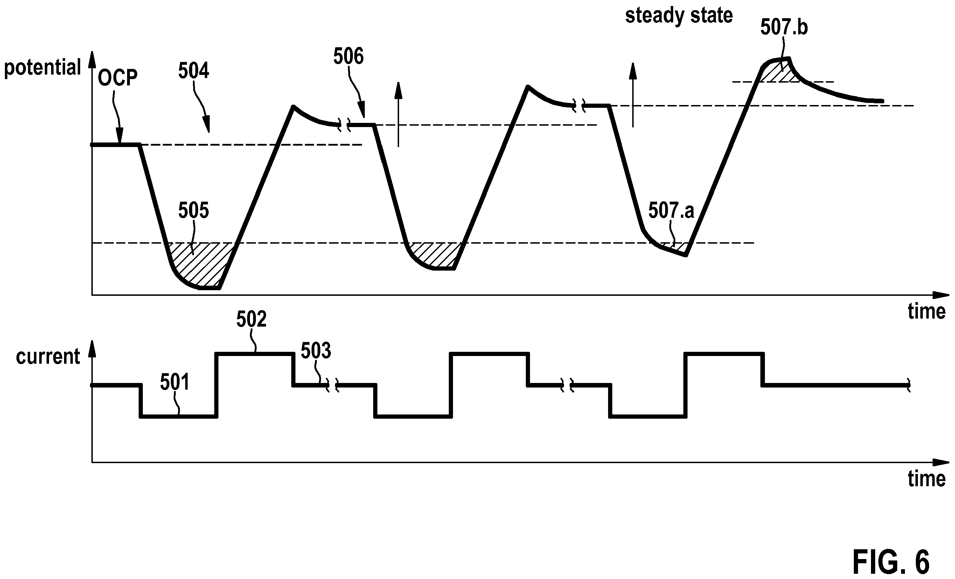

Regarding the automated charge balancing already indicated above, Figure 6 shows in a general manner the potential of a stimulating electrode when an active charge-balanced stimulation protocol is used in a high rate pulsing application. In this case, the stimulation pulse consists of a stimulation phase 501 (cathodic pulse) and a balance phase 502 (anodic pulse), followed by an open circuit phase 503 where no current is imposed by the pulse generator 104.

As it can be seen in Figure 6, the electrode potential begins from its open circuit potential (OCP) (measured against a suitable voltage reference electrode). During delivery of the first cathodic pulse 504, the electrode-tissue double layer reversibly charges and the electrode may begin to transfer charge into Faradaic reactions 505 as its potential moves negative. Since it is likely some irreversible charge transfer will occur during the stimulating phase 501, not all of the injected charge may go into charging the double layer under such situation. Hence, only a fraction of the cathodic charge of pulse 504 would be required during the anodic phase 502 to bring the potential back to OCP. If the anodic pulse 502 is balanced with the cathodic one 501 instead, as classically implemented in IPGs, the pre-pulse potential 506 of successive pulses moves positive until the same amount of charge is lost during the cathodic and anodic phases shaded areas 507.a and 507.b. If this occurs, the anodic Faradaic reaction 507.b may cause electrode corrosion. In the case of a platinum (Pt) electrode, for example, Pt oxide (PtO) may be formed and soluble Pt compounds generated when such PtO reacts in the chloride medium. A candidate toxic product is cisplatin $[PtCl_2(NH_3)_2]$.

The system 100 according to a preferred embodiment of the invention is thus particularly configured to deliver stimulation with a scheme that automatically adjusts the charges injected to maintain safe operation, and particularly to prevent voltage runaway in the DC blocking capacitors $C_i$ (where i denotes the electrode).

Now, during charge-imbalanced stimulation, the shift in pre-pulse potential may be either positive or negative of the OCP depending on the amount of imbalance. Hence, to be able to monitor electrode voltage drift and compensate for it during therapy (without interruption), the system 100 proposed particularly delivers the minimum charge imbalance necessary that guarantees at each active electrode both its associated DC blocking capacitor $C_i$ and double layer (which are in series) charge in the same direction. Under the proposed system 100 and method, the stimulating electrodes will charge in one direction whereas the return electrodes will charge in the opposite direction to allow compensating when certain voltage limits are reached.

The determination of the necessary imbalance may be performed prior to electrical stimulation of the target, for different patient postures, and depending on the stimulation frequency, by first independently cycling through each programmed stimulating electrode (to be used for electrical stimulation) and stimulating (as programmed for electrical stimulation) against a pseudo reference electrode instead. Such pseudo reference may preferably be the IPG casing 201a. Following, the system 100 cycles through all return electrodes except one which is forced to handle the current mismatches. During this "determination stage", parameters that measure the final programmed "unbalance" for each active electrode are saved, and the stimulation and return electrodes with the largest voltage drift, as well as the forced return electrode, selected for indirect monitoring during the actual electrical stimulation of the target (e.g. patient).

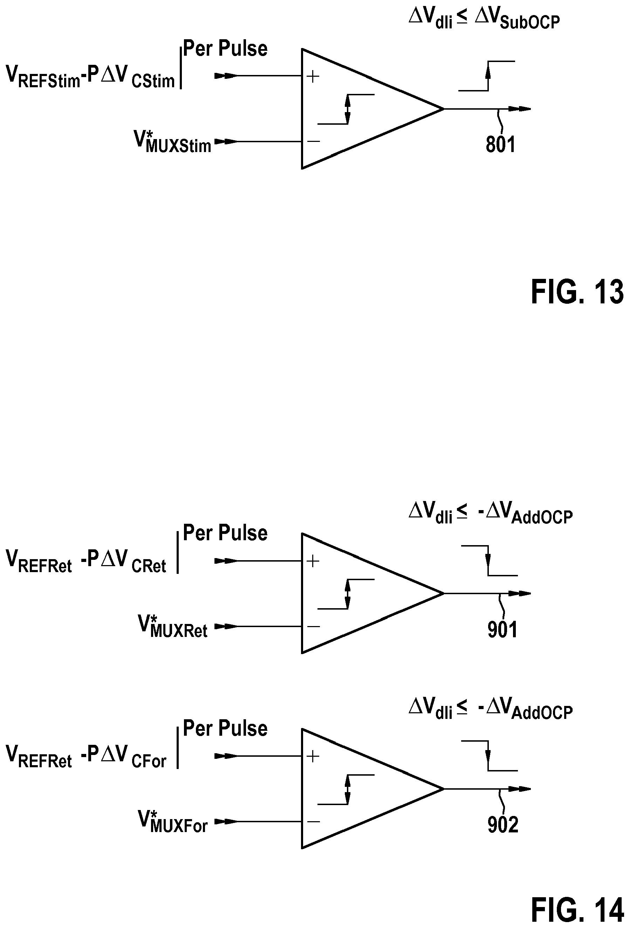

Once the determination stage is completed, electrical stimulation of the target is delivered as programmed and during the open circuit phases the accumulated electrode-tissue double-layer voltages (of the selected electrodes for monitoring) are particularly indirectly compared against variable reference voltages internally generated in the IPG 104. These comparators (cf. e.g. Figs. 13 to 14) preferably allow monitoring the stimulating and return electrode voltages (with the largest excursions), and the forced return electrode, between programmable limits without direct accessing such electrode voltages. Particularly, unlike in prior art, there are no measurements during electrical stimulation of the target, only comparison of voltages (e.g. a minimum of three voltages on the other side of the DC blocking capacitors for the selected electrodes) that indirectly assess the double-layer voltages accumulated. Advantageously, this approach reduces power consumption (no amplifiers are used during the actual electrical stimulation of the target), minimizes time to decide on the status of the electrodes, and requires no DC path from an electrode.

Particularly, in an embodiment, once a comparator triggers, correction phases take place to start moving the accumulated charges in the opposite directions. Particularly, these correction phases can either be performed by having a separate active phase during part of the open circuit phases or by adjusting successive balance phases.

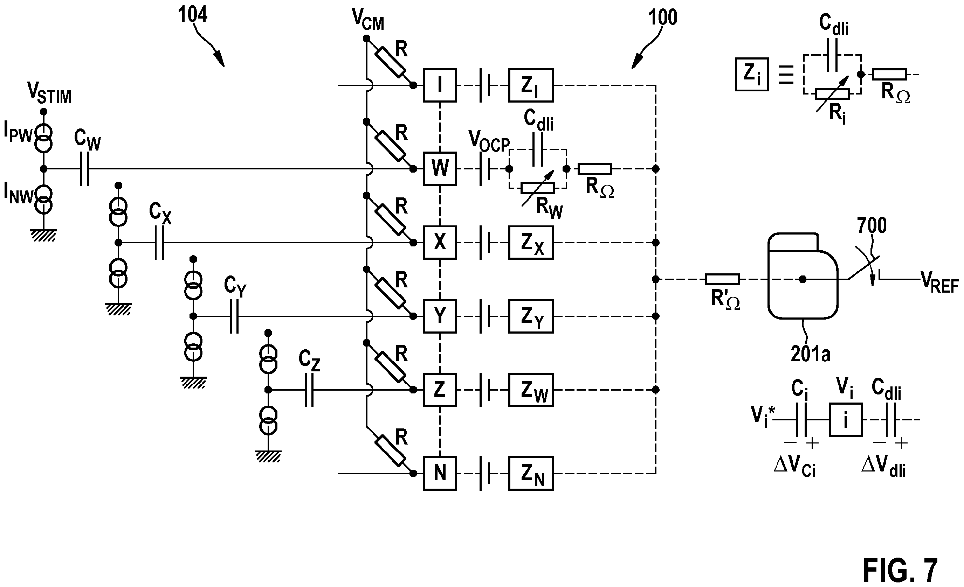

In the following a possible scheme for automated charge balancing is described in detail. To this end, Figure 7 shows a schematic representation of a front-end of an implantable pulse generator 104, which may be configured in an embodiment as an IPG of a spinal cord stimulator (SCS). Particularly, each electrode-tissue interface is modelled by an impedance $Z_i$ (Figure 7 top right) composed of the parallel of the electrode-tissue double-layer capacitance $C_{dli}$ with a variable resistor $R_i$ (representative of Faradaic reactions that may occur during stimulation/balancing), in series with $R_\Omega$ which represents the ohmic drop of the tissue electrolyte in the vicinity of an electrode.

Particularly, the IPG case 201a is made of a material that approximates a pseudo reference electrode (e.g. fractal Ir or TiN) and may comprise an effective area that makes its double-layer capacitance $C_{Case}$ (not shown) much larger than $C_{dli}$ (i = 1..N). The electrodes can be made, for example, of Pt, Pt/Ir, or fractal Ir. The open circuit potential (OCP) $V_{OCP}$ shown in Figure 7 is against the IPG case 201a when the latter is connected to an internally-generated voltage reference $V_{REF}$ via switch 700. Since all electrodes are of the same material and have similar areas, it can be considered they all have the same $V_{OCP}$ as reflected in Figure 7.

A similar $R'_\Omega$ represents the ohmic drop in the vicinity of the IPG case 201 a. The $R_\Omega$ and $R'_\Omega$ actual values are irrelevant as voltage monitoring for safe operation particularly occurs during the open circuit phases 503 when no current is imposed by the IPG 104 (or it can be neglected for the purpose of the analysis). The voltage $V_{STIM}$ in Figure 7 is particularly programmed in an embodiment with the required minimum overhead for steady-state stimulation.

$C_i$ represent the DC blocking capacitor associated with each electrode (i = 1..N, only $C_W$ to $C_Z$ shown in Figure 7) which are nominally all equal. It can also be guaranteed by design that $C_{case}$ results much larger than $C_i$. Typically, the $C_i$ value used in IPGs is in the order of 10 $\mu$F. $C_{dli}$, on the other hand, for an SCS Pt electrode, for example, has a value in the order of 12.5 $\mu$F. Fractal Ir coated electrodes will present a higher $C_{dli}$. In the case of nerve cuff electrodes, $C_{dli}$ may be lower. The present invention, however, assumes no particular relative values between $C_{dli}$ and $C_i$ for the purpose of implementing safe electrical stimulation.

Components R in Figure 7 are bleeding resistors (e.g. hundreds of k$\Omega$), placed in star configuration, typically utilized in IPG's front-ends for passive charge neutrality. In a preferred embodiment, the present invention re-utilizes such network for the purpose of implementing safe stimulation as it will be described below.

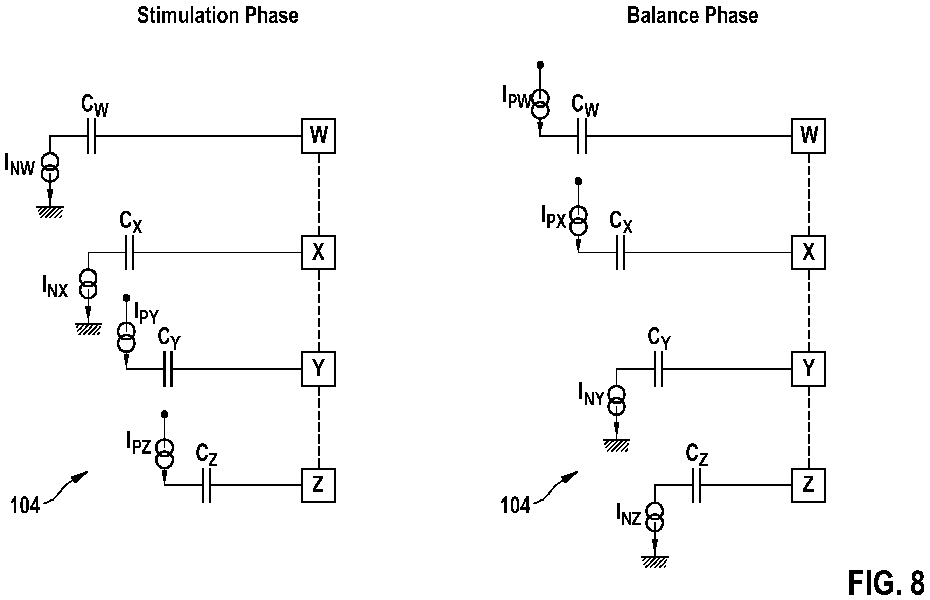

It is now assumed without losing generality that electrodes W, X, Y, Z are active during delivery of electrical stimulation to the target and that, for example, W, X are the stimulating electrodes and Y, Z the return electrodes of the stimulation phases as shown in Figure 8. During the stimulation phase, sinking currents $I_{NW}$ and $I_{NX}$ will flow through electrodes W and X respectively, whereas sourcing currents $I_{PY}$ and $I_{PZ}$ flow through electrodes Y and Z. Currents are particularly programmed so the total cathodic current equals the total anodic current, i.e. in this case

$$I_{NW} + I_{NX} = I_{PY} + I_{PZ} \qquad (1)$$

Assuming the sourcing currents (those from $V_{STIM}$) present larger output impedance than the sinking currents (those to ground), the latter will accommodate their real values to satisfy eq. (1). In a preferred embodiment, the system of the present invention adjusts the output impedance of the current source associated with at least one of the return electrodes in the stimulation phase (e.g. contact Z) to implement tissue and electrode safe operation. This is further described later on.

An active balance phase is opposite as shown in Figure 8, i.e. currents $I_{PW}$ and $I_{PX}$ flow instead through electrodes W and X respectively, whereas currents $I_{NY}$ and $I_{NZ}$ will flow through electrodes Y and Z.

For the actual electrical stimulation of the target (therapy), currents $I_{NW}$, $I_{NX}$, $I_{PY}$, and $I_{PZ}$, the stimulation phase pulse width ($PW_{Stim}$, common to all), the balance phase pulse width ($PW_{Bal}$, common to all), the inter phases delay (i.e. the time between the end of a stimulation pulse and the start of the associated balancing pulse), and the stimulation frequency are typically selectable and programmable in an IPG 104. For high pulsing rates, and for closed-loop neurostimulation based on neural response, $PW_{Bal}$ is preferably selected equal to $PW_{Stim}$ and programmed as a single parameter pulse width (PW). The balance phase currents $I_{PW}$, $I_{PX}$, $I_{NY}$, and $I_{NZ}$ can be the unknowns the system may adjust to implement safe stimulation without therapy interruption.

Hence, in a preferred embodiment of the present invention, the balance phase currents are automatically determined for safe operation.

For safe tissue and electrode stimulation, the accumulated voltage of the equivalent double-layer capacitances ($\Delta V_{dli}$ where i = W, X, Y, Z in the example) shall remain within a safe window. With the sign shown in Figure 7 (bottom right), this translates into

$$-\Delta V_{AddOCP} \leq \Delta V_{dli} \leq \Delta V_{SubOCP} \qquad (2)$$

where $\Delta V_{SubOCP}$ and $\Delta V_{AddOCP}$ limit the excursion of the electrode voltage in the negative and positive direction with respect to its open circuit potential (OCP) respectively. The limit values may be determined via in-vitro experiments using a suitable electrolyte (and confirmed in-vivo) and programmed in the IPG 104. In a preferred embodiment, the window is symmetrical and a few hundred mV wide (e.g. ± 100 mV).

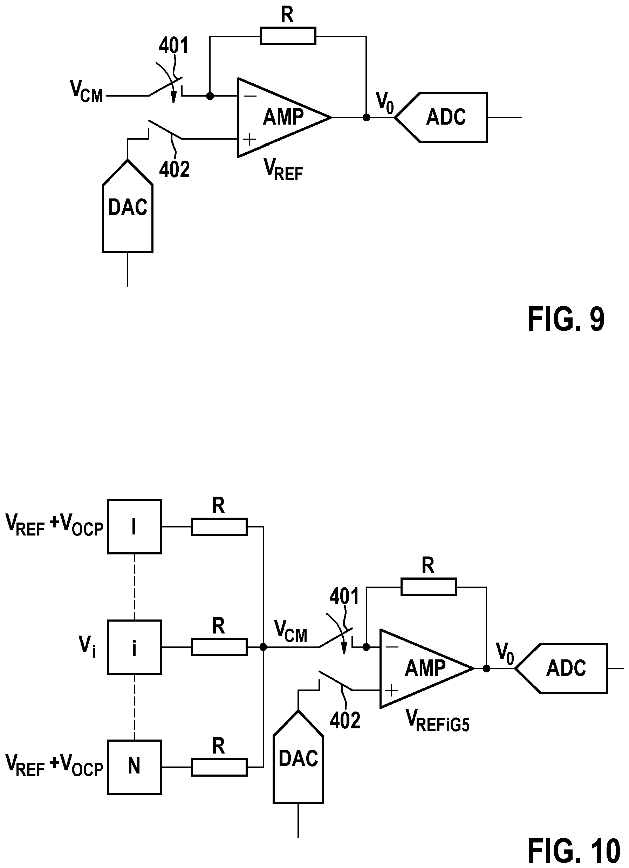

A preferred embodiment for safe stimulation is the following: prior to delivery of the actual electrical stimulation to the target and particularly for different patient postures, the IPG 104 first estimates $V_{OCP}$. To do so, it is configured to measure the common point $V_{CM}$ of the bleeding resistor network R (see Figure 7), preferably via the circuit of Figure 9 (switches 401 and 402 are closed) when the digital-to-analog converter block (DAC) outputs a reference voltage $V_{REF}$ and the IPG case 201 is connected to such voltage. In such case, the output Vo of the amplifier AMP equals

$$Vo = -N\,V_{OCP} + V_{REF} \qquad (3)$$

which is preferably digitized via the analog-to-digital converter block (ADC) and the $V_{OCP}$ calculated and stored in the IPG 104 (N is typically 2, 4, 8, 16, or 32 in a neuro stimulator so digital division is straightforward). Switches 401 and 402 are particularly designed with negligible charge injection and on-resistance compared to R. The amplifier AMP offset is particularly also negligible for the purpose of determining $V_{OCP}$. The resistor R in the feedback of amplifier AMP is preferably matched with the resistors R of Figure 7 to reduce measurement error.

Now, as mentioned before, to be able to monitor voltage drift and compensate for it, the system 100 is preferably configured to deliver the minimum charge imbalance that guarantees (at each electrode) that both $C_i$ and $C_{dli}$ charge in the same direction. The stimulating electrodes (W and X in the example) and the return electrodes (Y and Z in the example) of the stimulation phase will charge in opposite directions to allow compensating once a limit given by conditions (2) is reached.

The determination stage prior to the stimulation stage (where the actual electrical stimulation of the target takes place) may proceed as follows:

The system 100 preferably first cycles through each stimulating electrode independently (W and X in the example) and injects M (M = 2, 4, 8,..) "balanced as-programmed pulses" leading to the ramp envelope (i.e. $I_{Pi}$ is automatically programmed equal to $I_{Ni}$) against the IPG case 201 a (return electrode in the determination stage). The balance will then only be limited by the current matching between the real $I_{Ni}$ and real $I_{Pi}$ (which is typically calibrated for and a few percent apart). M may be selected to improve accuracy of the calculations detailed below. In between the cycling of electrodes W and X (in the example), a complete passive balance phase for electrode W and IPG case 201a (with hardware not shown in Figure 7) is preferably performed to guarantee an electrical-neutral system before cycling the next electrode (X in the example). The same procedure applies when two or more stimulating electrodes are utilized instead for therapy.

For the determination stage, $V_{STIM}$ may be re-programmed with different values to mimic the actual varying voltage that will appear across each current source/sink during therapy. For electrode W, for example, $V_{STIM}$ may be temporarily re-programmed (during the determination stage) with a value equal to

$$V_{DSn} + R_{W2casemax} * I_{NWmax} + (I_{NWmax} * PW) / C_{WdlWmin}$$

where $V_{DSn}$ is a "safe" compliance voltage required for the current sinks to operate, $R_{W2casemax}$ is the measured impedance between electrode W and the IPG case 201 a increased by the measurement error, $I_{NWmax}$ is the stimulation current through electrode W increased by the allowable error, PW is the stimulation pulse width, and $C_{WdlWmin}$ is the measured series capacitor $C_W$, $C_{dlW}$ decreased by the measurement error. It is particularly assumed that $V_{DSn}$ has enough overhead to accommodate the maximum steady-state accumulated voltage on $C_W$ and $C_{dlW}$ for the determination stage to properly operate under such reduced $V_{STIM}$. Given each electrode is much smaller than the IPG case 201a, this setup emulates what each electrode will see under a multi-current therapy setup.

After the M determination pulses in the stimulating electrode "i" (i = W or X in the example), connecting again the circuit of Figure 9 and the IPG case 201 a results in the circuit of Figure 10. All electrodes, except the cycled "i" (which was active), still present a voltage equal to ($V_{REF} + V_{OCP}$). Programming now the DAC block reference to a voltage $V_{REFFIG5}$ equal to such ($V_{REF} + V_{OCP}$) results in

$$Vo = \Delta V_{dli} + V_{REFFIG5} \qquad (4)$$

as

$$V_i = -\Delta V_{dli} + V_{REF} + V_{OCP} = -\Delta V_{dli} + V_{REFFIG5} \qquad (5)$$

(see Figure 7 for the defined sign of $\Delta V_{dli}$).

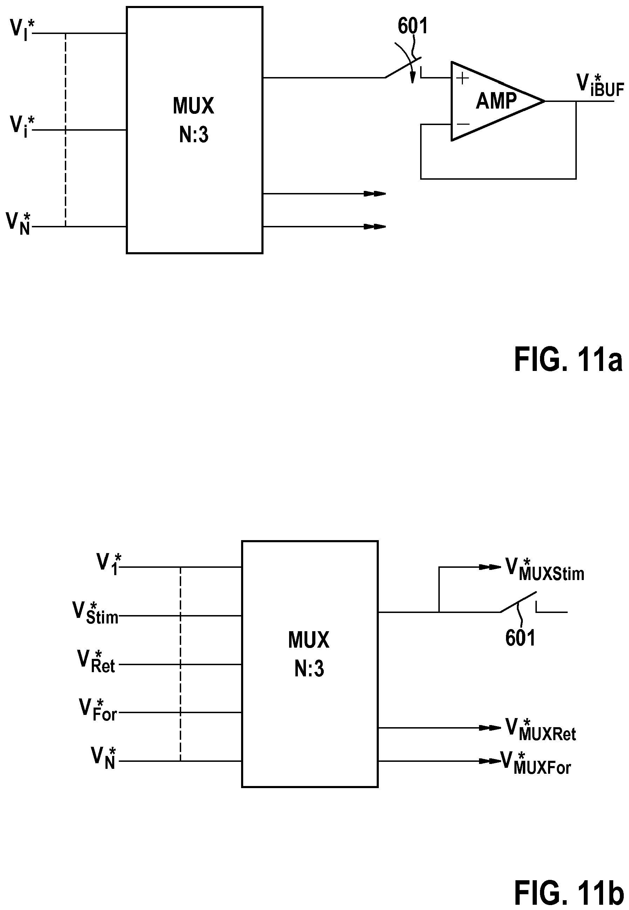

At the same time, the system 100 particularly also measures $V^*_i$ which is the voltage at the other terminal of the DC blocking capacitor $C_i$ of active electrode "i" cycled (see Figure 7 bottom right). This is measured via the N to 3 multiplexer (MUX) block, switch 601 and buffer (AMP) shown in Figure 11.a ($V^*_{iBUF}$ is the output signal).

From $V_i$ determined above (see eq. (5)) and $V^*_{iBUF}$, the accumulated voltage $\Delta V_{Ci}$ (from current mismatches) on the blocking capacitor $C_i$ can be calculated as ($V_i - V^*_{iBUF}$) (see Figure 7 for the sign of $\Delta V_{Ci}$ as measured).

If both $\Delta V_{dli}$ and $\Delta V_{Ci}$ resulted positive, the balance phase for the cycled electrode "i" can be left as programmed for the determination stage. No adjustments are necessary as the positive voltages indicate the mismatch in the real $I_{Ni}$ and real $I_{Pi}$ is causing the balancing charge to be less than the stimulation charge. The misbalance current $I_{Diffi}$, i.e. real $I_{Ni}$ - real $I_{Pi}$, can be estimated to be at least

$$I_{Diffi} = C_{imin} (\Delta V_{Ci}) / (M\,PW) \qquad (i = W\ or\ X\ in\ the\ example) \qquad (6)$$

where $C_{imin}$ is the minimum value of the DC blocking capacitor $C_i$, $\Delta V_{Ci}$ is the above measured accumulated voltage, and PW is the programmed pulse width as defined before.

If $\Delta V_{dli}$ is negative, on the other hand, this implies the electrode "i" potential would be moving positively pulse after pulse so less balancing charge is required to avoid this situation. In a preferred embodiment, the required reduction is determined as follows:

A prior impedance measurement allows estimating $C_{dli}$ for the electrode "i" under consideration (either W or X in the example), with a certain error, so the current $I_{Lessi}$ required to be subtracted from the automatically selected $I_{Pi}$ in this case can be calculated as follows

$$I_{Lessi} = C_{dlimax} (-\Delta V_{dli}) / (M\,PW) \qquad (i = W\ or\ X\ or\ none\ in\ the\ example) \qquad (7)$$

where $C_{dlimax}$ is the measured $C_{dli}$ with the maximum added error, $\Delta V_{dli}$ is the above measured accumulated double-layer voltage (see Figure 7), and PW is the programmed pulse width as defined before.

A lookup table can be implemented in the IPG 104 to determine each $I_{Diffi}$, $I_{Lessi}$ based on the corresponding C, $\Delta V$, and (M PW).

For those electrodes with $\Delta V_{dli}$ negative, $I_{Pi}$ will then be automatically re-programmed equal to

$$new\ I_{Pi} = old\ I_{Pi} - I_{Lessi} \qquad (i = W\ or\ X\ or\ none\ in\ the\ example) \qquad (8)$$

where $I_{Lessi}$ is the current estimated above.

Having a positive $\Delta V_{dli}$ and a negative $\Delta V_{Ci}$ is not possible as the latter implies the automatically programmed $I_{Pi}$ was larger than the selected $I_{Ni}$ (by mismatch) which will always result in a negative $\Delta V_{dli}$ regardless of whether Faradaic reactions were present or not during the stimulation phase.

After initially cycling through all stimulating electrodes, a new set of M pulses, with the modified balance phase, is preferably injected for the stimulating electrodes that required $I_{Pi}$ adjustment, their new $I_{Diffi}$, estimated and stored, and confirming both $C_i$ and $C_{dli}$ accumulated charge in the same direction.

At the end of this process, all stimulating electrodes "i" (W and X in the example) will in theory satisfy

$$real\ I_{Pi} = real\ I_{Ni} - I_{Diffi} \qquad (9)$$

The lowest value among the estimated $I_{Diffi}$ from all stimulating electrodes (W and X in the example) is stored in the IPG 104 as $I_{MinDiff}$. Alternatively, the $\Sigma\ I_{Diffi}$, divided by the number of return electrodes in the stimulation phase is stored instead as $I_{MinDiff}$.

Hence, in this way, $\Delta V_{Ci}$ for the stimulating electrodes (W and X in the example) will have the same positive sign as $\Delta V_{dli}$ as the real $I_{Pi}$ for therapy is guaranteed to be less than $I_{Ni}$.

However, $I_{Pi}$ was determined with only one electrode active. For the same $I_{Pi}$ to flow during therapy where all programmed electrodes are active simultaneously, at least a return electrode in the stimulation phase (e.g. Z considering without losing generality that $I_{PZ}$ is the smallest return current amplitude of the stimulation phase) needs to be forced to present lower impedance than the sinking currents so the $I_{Ni}$ currents get properly established.

On the other hand, in the case of the return electrodes of the stimulation phase, except for the one forced to have lower impedance (i.e. Z in the example), the balance phase currents are preferably automatically programmed equal to

$$I_{Ni} = I_{Pi} - I_{MinDiff} \qquad \text{(i = Y in the example)} \qquad (10)$$

where $I_{MinDif}$ was stored in the IPG 104 as described before.

The system 100 will then preferably cycle independently through each return electrode of the stimulation phase except the forced one (only Y in the example), injecting again M (M = 2, 4, 8,..) with the selected $I_{Pi}$ and automatically-programmed $I_{Ni}$ (see eq. (10)) against the IPG case 201a (return electrode in this stage).

After the M pulses, the difference between the real $I_{Pi}$ and real $I_{Ni}$ can be estimated as follows

$$(\text{real } I_{Pi} - \text{real } I_{Ni}) = C_{imax} (-\Delta V_{Ci}) / (M\ PW) \quad (11)$$

The system 100 then verifies

$$0 < (\text{real } I_{Pi} - \text{real } I_{Ni}) \leq I_{MinDiff} \qquad (12)$$

and (real $I_{Pi}$ - real $I_{Ni}$) is defined as $\Delta I_i$.

If condition (12) is not satisfied, the system 100 can automatically adjust $I_{Ni}$ until condition (12) is satisfied as $I_{Pi}$ is the programmable parameter of the stimulation phase.

The remaining of the sourcing/sinking currents of the stimulation/balance phase will circulate through the forced electrode (Z in the example).

In this way, the stimulating and return electrodes are charging in opposite directions allowing for compensation when one of the conditions (2) is reached.

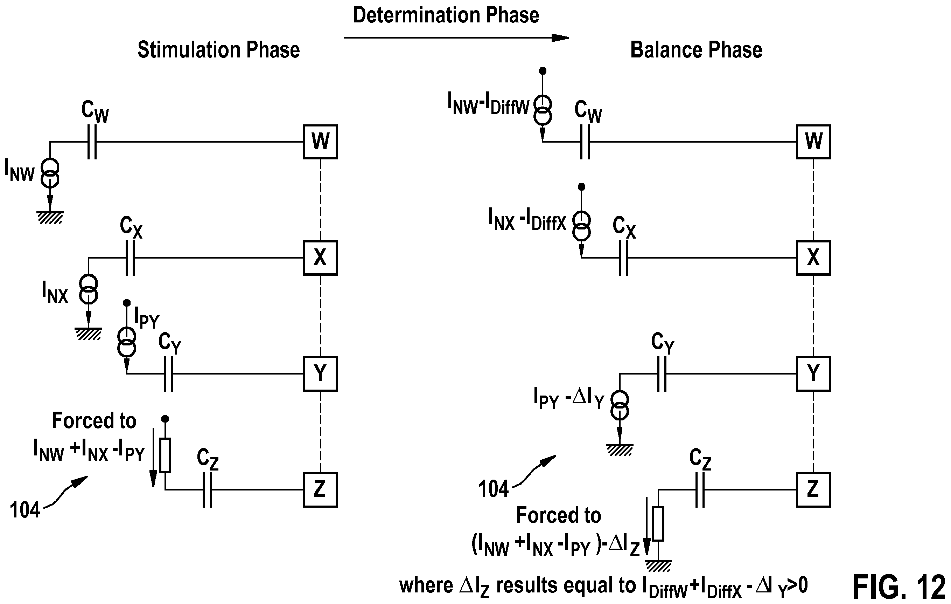

Summarizing, the stimulation and balance phases (post determination stage) in the example being described are shown in Figure 12. Programmable resistors, instead of a current source or sink, can be used to force electrode Z (forming the forced return electrode in the example) to present lower impedance in both the stimulation and balance phases. Considering the stimulation phase, for example, such resistor can be programmed equal to $\{V_{SDp} /$

$$I_{pZmin} + [(I_{PYmax} / I_{Pzmin}) / C_{YdlYmin} - 1 / C_{ZdlZmax}] * PW + (I_{PYmax} / I_{Pzmin} * R_{Y2allEmax} -$$

$$R_{Z2allEmin})\}$$

where $V_{SDp}$ is a "safe" compliance voltage required for the current sources to operate, min and max subscripts represent the respective parameters with added or subtracted errors, and $R_{i2allE}$ (i = Y, Z in the example) is the impedance of electrode "i" against all other electrodes tied together. Compliance voltage monitoring across active sink and sourcing currents during the actual electrical stimulation of the target permit confirming the selected resistor is appropriate. If two or more return electrodes are programmed, electrode Z represents the electrode with the smallest programmed current.

As a final step of the determination stage, a new set of M pulses, with the determined balance phase, is preferably injected next for all active electrodes (i.e. both the stimulating and returns) this time, except for the forced one (Z in the example). The parameters $\Delta V_{dli}$ and $\Delta V_{Ci}|^{Per\ Pulse}$ for each electrode are now determined, the latter particularly as the measured $\Delta V_{Ci}/M$ for the selected stimulating and return electrodes, and particularly as

$$[(\Sigma\ I_{Diffi} - \Sigma\ \Delta I_i) * PW] / C_{imin}$$

for the forced electrode (Z in the example), and the former values digitized and stored in the IPG 104. For the forced electrode (Z in the example), a new lookup table can be implemented to determine $\Delta V_{CFor}|^{Per\ Pulse}$ (accumulated per-stimulation pulse voltage in the DC blocking capacitor associated with the forced electrode, Z in the example).

The system 100 will preferably select and monitor (during delivery of the electrical stimulation to the target) the stimulating and return electrodes that presented the largest $|\Delta V_{dli}|$. It will also monitor the forced electrode (Z in the example). The voltages $V^*_{Stim}$, $V^*_{Ret}$, and $V^*_{For}$ (see Figure 11.b) of the selected stimulating, return, and forced electrodes will particularly be connected to $V^*_{MUXStim}$, $V^*_{MUxRet}$, and $V^*_{MUXFor}$ respectively via the MUX. The circuitry of Figure 9 (Figure 10), except for the DAC, and the AMP of Figure 11.a are not needed for the actual electrical stimulation of the target so they may be turned off/disconnected to reduce power consumption.

In an alternative embodiment, all voltages of the participating active electrodes may be monitored instead.

As mentioned before, during electrical stimulation of the target, the system shall guarantee

$$-\Delta V_{AddOCP} \leq \Delta V_{dli} \leq \Delta V_{SubOCP} \quad (i = 1..N)\ (13)\ (same\ as\ eq.\ (2))$$

Now, during an open circuit phase (no current is imposed by the IPG 104), if the IPG case 201a is connected to $V_{REF}$, in particular one has for the monitored voltages that

$$V_{REF} + V_{OCP} - \Delta V_{dlOutput} - \Delta V_{COutput} - V^*_{MUXOutput} = 0 \qquad (14)$$

(with the sign shown in Figure 7) where Output is either Stim, Ret or For. Eq. (13) can be re-written as

$$\Delta V_{dlOutput} = V_{REF} + V_{OCP} - \Delta V_{COutput} - V^*_{MUXOutput} \qquad (15)$$

At the same time, after P stimulation pulses,

$$\Delta V_{COutput} = \Sigma_{1\ to\ P}\ \Delta V_{COutput}|^{Per\ Pulse} = P\ \Delta V_{COutput}|^{Per\ Pulse} \qquad (16)$$

where the parameter $\Delta V_{Cutput}|^{Per\ Pulse}$ was previously digitized and internally stored in the IPG 104 in the final step of the determination stage.

Hence from (13), (15) and (16), for the monitored voltages we have

$$-\Delta V_{AddOCP} \leq V_{REF} + V_{OCP} - P\ \Delta V_{COutput}|^{Per\ Pulse} - V^*_{MUXOutput} \leq \Delta V_{SubOCP} \qquad (17)$$

Conditions (17) can be individually re-written as

$$V^*_{MUXStim} \geq V_{REF} + V_{OCP} - \Delta V_{SubOCP} - P\ \Delta V_{CStim}|^{Per\ Pulse} \qquad (18.a)$$

$$V^*_{MUXRet} \leq V_{REF} + V_{OCP} + \Delta V_{AddOCP} - P\ \Delta V_{CRet}|^{Per\ Pulse} \qquad (18.b)$$

$$V^*_{MUXFor} \leq V_{REF} + V_{OCP} + \Delta V_{AddOCP} - P\ \Delta V_{CFor}|^{Per\ Pulse} \qquad (18.c)$$

It is worth pointing out that $\Delta V_{CRet}|^{Per\ Pulse}$ and $\Delta V_{CFor}|^{Per\ Pulse}$ in conditions 18.b and 18.c are negative so they add to the value on the right of such inequalities.

Conditions 18 can re-written as

$$V^*_{MUXStim} \geq V_{REFStim} - P\ \Delta V_{CStim}|^{Per\ Pulse} \qquad (19.a)$$

$$V^*_{MUXRet} \leq V_{REFRet} - P \ \Delta V_{CRet}|^{Per \ Pulse} \tag{19.b}$$

$$V^*_{MUXFor} \leq V_{REFRet} - P \ \Delta V_{CFor}|^{Per \ Pulse} \tag{19.c}$$

where $VR_{EFStim}$ and $V_{REFRet}$ are fixed voltages equal to $(V_{REF} + V_{OCP} - AV_{SubOCP})$ and $(V_{REF} + V_{OCP} + \Delta V_{AddOCP})$ respectively.

In a preferred embodiment, condition 19.a is implemented by the comparator of Figure 13 where an extra DAC block generates a variable reference that subtracts the stored $\Delta V_{C\text{-}Stim}|^{Per \ Pulse}$ after each stimulation phase from the internally calculated fixed voltage $V_{REF\text{-}Stim}$, for comparison following the end of the balance phase of pulse P and before the beginning of the next stimulation phase.

Similarly, conditions (19.b) and (19.c) are implemented by the comparators of Figure 14, where a third and fourth internal DAC generate the variable comparison voltages.

If a comparator of Figure 13 or Figure 14 triggers (i.e. outputs 801, 901, or 902 change logic value), after P pulses (a counter is kept in the IPG 104), the corresponding double-layer capacitance and blocking capacitor of the monitored electrode will be discharged.

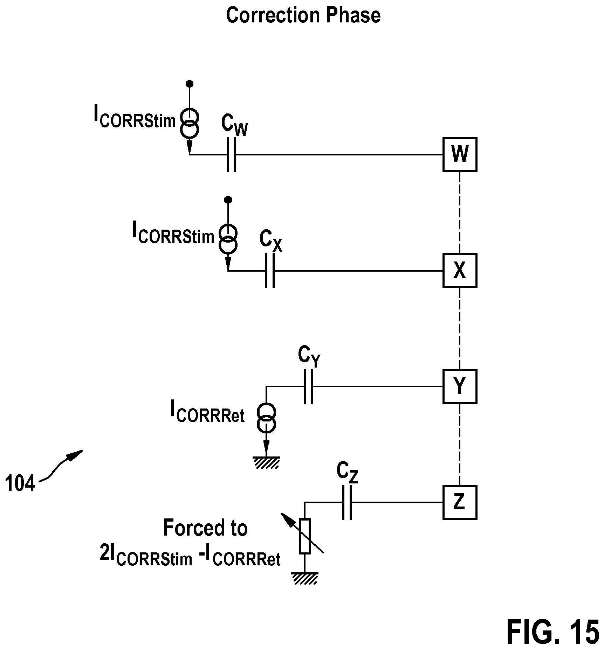

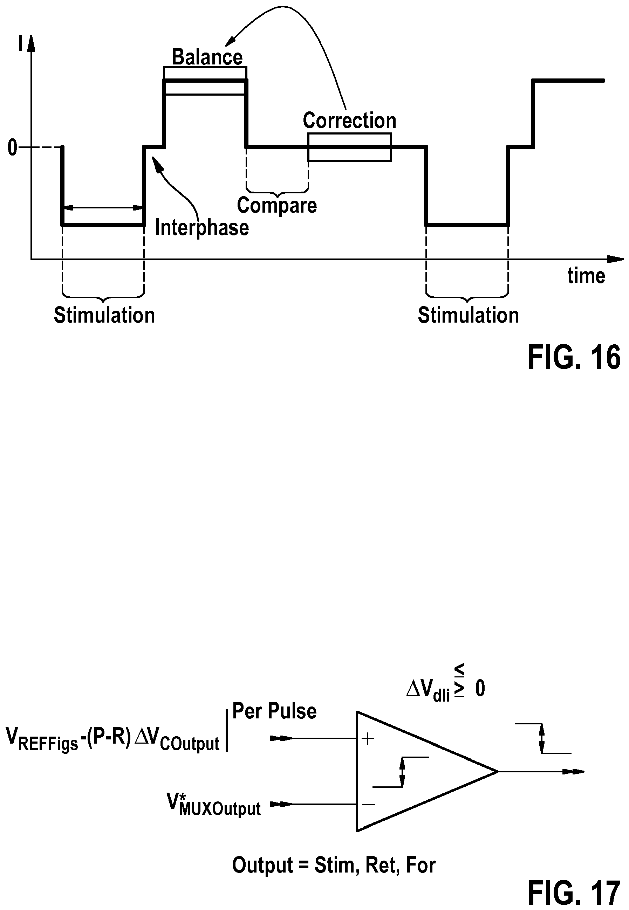

To do so, in a preferred embodiment, a correction phase is implemented as shown in Figure 15 for example. Particularly, a single correction current $I_{CORR}$ will be forced to circulate which will result in real currents $I_{CORRStim}$ for the stimulating electrodes (W and X in the example) and $I_{CORRRet}$ for the return electrodes (only Y in the example). The forced electrode (Z in the example) will handle the difference between the correcting currents. Such correction phases particularly take place following the compare phases (where conditions 18 are evaluated) as shown in Figure 16. It is also possible to stagger the compare and correction phases so they occur in subsequent pulses. In an alternative embodiment, the correction phase is part of the balance phase (e.g. as sketched) where the currents of the latter phase are adjusted accordingly, so as to reduce or cancel the respective accumulated double layer voltage.

In a preferred embodiment, current $I_{CORR}$ is programmed equal to two times $I_{MinDiff}$.

Since it is unknown which capacitor has accumulated more charge, $C_{Output}$ or $C_{dlOutput}$ for the active electrode whose $V^*_{MUXOtput}$ triggered a comparator, the system 100 needs to deliver up to P pulses and stop if $\Delta V_{dlOutput}$ reaches zero voltage ($\Delta V_{COutput}$ will still be positive or negative depending on the electrode). This avoids inverting the charging conditions of the stimulating and return electrodes. Hence, during the injection of the correction phases, the system will make sure the following conditions are satisfied

$$\Delta V_{dlStim} = V_{REF} + V_{OCP} - \Delta V_{CStim} - V^*_{MUXStim} \geq 0 \tag{20.a}$$

$$\Delta V_{dlRet} = V_{REF} + V_{OCP} - \Delta V_{CRet} - V^*_{MUXRet} \leq 0 \tag{20.b}$$

$$\Delta V_{dlFor} = V_{REF} + V_{OCP} - \Delta V_{CFor} - V^*_{MUXFor} \leq 0 \tag{20.c}$$

or re-written as

$$V^*_{MUXStim} \leq V_{REF} + V_{OCP} - \Delta V_{CStim} \tag{21.a}$$

$$V^*_{MUXRet} \geq V_{REF} + V_{OCP} - \Delta V_{CRet} \tag{21.b}$$

$$V^*_{MUXFor} \geq V_{REF} + V_{OCP} - \Delta V_{CFor} \tag{21.c}$$

or re-written as

$$V^*_{MUXStim} \leq V_{REFFIG5} - \Delta V_{CStim} \tag{22.a}$$

$$V^*_{MUXRet} \geq V_{REFFIG5} - \Delta V_{CRet} \tag{22.b}$$

$$V^*_{MUXFor} \geq V_{REFFIG5} - \Delta V_{CFor} \tag{22.c}$$

or re-written as

$$V^*_{MUXStim} \leq V_{REFFIG5} - (P-R) \Delta V_{Cstim}\big|^{Per\ Pulse} \tag{23.a}$$

$$V^*_{MUXStim} \geq V_{REFFIG5} - (P-R) \Delta V_{CRet}\big|^{Per\ Pulse} \tag{23.b}$$

$$V^*_{MUXStim} \geq V_{REFFIG5} - (P-R) \Delta V_{CFor}\big|^{Per\ Pulse} \tag{23.c}$$

After R correction phase pulses (R $\leq$ P), R $\Delta V_{COutput}\big|^{Per\ Pulse}$ has been subtracted from the accumulated $\Delta V_{COutput}$ (given $I_{CORR}$ equals $2I_{MinDiff}$) so $V^*_{MUXOutput}$ (of the triggered comparator) needs to be compared against a variable reference equal to

$$V_{REFFIG5} - (P-R)\Delta V_{COutput}\big|^{Per\ Pulse} \text{ as shown in Figure 12.}$$

If the comparator in Figure 17 is triggered, or R equals P, the correction phase is stopped and actual electrical stimulation of the target as per Figure 12 resumed.

## Claims

1. Method for generating a premodulated interferential current using a pulse generator (104) having at least one electrode (102.a, 102.b), wherein the pulse generator is configured to generate a premodulated current (300) which is output using the at least one electrode (102.a, 102.b), wherein said current (300) comprises a train of biphasic pulses (301) having a frequency ($f_{train}$), wherein each biphasic pulse (301) comprises a stimulating phase (304) and a balancing phase (305), and wherein said premodulated current (300) comprises an amplitude modulation envelope (307, 308) having an envelope beat frequency ($f_{beat}$) being smaller than said frequency ($f_{train}$) of the biphasic pulses (301), wherein said modulation envelope (307, 308) is generated in the pulse generator (104).

2. Method according to claim 1, wherein said phases (304, 305) are rectangular pulses, wherein particularly the stimulating phase (304) of the respective biphasic pulse (301) is separated from the succeeding balancing phase (305) of the respective biphasic pulse (301) by an adjustable interphase delay ($T_D$).

3. Method according to claim 1 or 2, wherein the modulation envelope (307, 308) of said premodulated current (300) ramps up to a maximal amplitude ($I_{MAX}$) and then ramps down to a minimal amplitude.

4. Method according to one of the preceding claims, wherein the envelope beat frequency ($f_{beat}$) is automatically varied by the pulse generator (104) and/or manually by a user.

5. Method according to claim 4, wherein the envelope beat frequency ($f_{beat}$) is changed by either automatically removing pulses (301) from the train (300) or automatically adding pulses to the train (300).

6. Method according to claim 5, wherein pulses (301) are automatically added to the train (300) or automatically removed from the train (300) in a manner that translates into a gradual sweep of the envelope beat frequency ($f_{beat}$), particularly in the form of a triangular pattern between a lower envelope beat frequency ($f_{beatL}$) and a higher envelope beat frequency ($f_{beatH}$).

7. Method according to one of the preceding claims, wherein premodulated currents (300) are delivered simultaneously using multiple electrodes (4a, 4b, 5a, 5b, 3a, 3b) to perform current steering.

8. Method according to one of the preceding claims, wherein for automatic charge balancing during a stimulation stage, in which a premodulated current (300) is applied to a target, stimulating phases (304, $I_{Ni}$) and balancing phases (305, $I_{Pi}$) for the respective electrode are automatically programmed in a determination stage preceding said stimulation stage such that the difference (real $I_{Pi}$ - real $I_{Ni}$) between the circulating balancing current pulse (305, $I_{Pi}$) minus the respective stimulation current pulse phase (304, $I_{Ni}$) through the respective electrode satisfy:

- For at least one stimulating electrode, the difference equals an automatically determined positive value which may be different for different electrodes;
- For at least one returning electrode, the difference is positive and smaller or equal than the minimum among the difference values for the stimulating electrodes;
- For each electrode, both a DC blocking capacitor ($C_i$) which couples the respective electrode to a current source, a current sink, or a voltage, and a double layer capacitance ($C_{dli}$), wherein the double layer capacitance ($C_{dli}$) is formed by each electrode and the respective adjacent material, charge in the same direction and opposite for stimulating and returning electrodes, and wherein

- in said stimulation stage at least one of the electrodes is monitored, wherein, when a voltage ($\Delta V_{dli}$) accumulated at the double layer of the at least one monitored electrode crosses pre-defined thresholds ($-\Delta V_{AddOCP}$, $\Delta V_{SubOCP}$), correction currents ($I_{CORRStim}$, $I_{CORRRet}$) are generated and applied that reduce or cancel said accumulated voltages ($\Delta V_{dli}$).

9. System (100) for generating a premodulated interferential current, wherein the system comprises an implantable pulse generator (104) having at least one electrode (102.a, a02.b), wherein the pulse generator (104) is configured to generate a premodulated current (300) using at least one electrode (102.a, 102.b), wherein said current (300) comprises a train of biphasic pulses (301) having a frequency ($f_{train}$), wherein each biphasic pulse (301) comprises a stimulating phase (304) and a balancing phase (305), and wherein said premodulated current (300) comprises an amplitude modulation envelope (307, 308) having an envelope beat frequency ($f_{beat}$) being smaller than said frequency ($f_{train}$).

10. System according to claim 9, wherein said phases (304, 305) are rectangular pulses, wherein particularly the stimulating phase (304) of the respective biphasic pulse (301) is separated from the succeeding balancing phase (305) of the respective biphasic pulse (301) by an adjustable interphase delay ($T_D$).

11. System according to claim 9 or 10, wherein the pulse generator (104) is configured to generate said premodulated current (300) such that the modulation envelope (307, 308) of said premodulated current (300) ramps up to a maximal amplitude ($I_{MAX}$) and then ramps down to a minimal amplitude.

12. System according to one of the claims 9 to 11, wherein the pulse generator (104) is configured to automatically vary the envelope beat frequency ($f_{beat}$) or comprises a means configured for manually adjusting the envelope beat frequency ($f_{beat}$).

13. System according to claim 12, wherein the pulse generator (104) is configured to change the envelope beat frequency ($f_{beat}$) by either removing pulses (301) from the train (300) or by adding pulses (301) to the train (300).

14. System according to claim 13, wherein the pulse generator (104) is configured to add pulses (301) to the train (300) or to automatically remove pulses (301) from the train (300) in a manner that translates into a gradual sweep of the envelope beat frequency ($f_{beat}$), particularly in the form of a triangular pattern between a lower envelope beat frequency ($f_{beatL}$) and a higher envelope beat frequency ($f_{beatH}$).

15. System according to one of the claims 9 to 14, wherein the pulse generator (104) is configured to simultaneously deliver premodulated currents (300) using multiple electrodes (4a, 4b, 5a, 5b, 3a, 3b) to perform current steering.

16. System according to one of the claims 9 to 15, wherein for automatic charge balancing during a stimulation stage, in which a premodulated current (300) is applied to a target, the system (100) is configured to automatically program stimulating phases (304, $I_{Ni}$) and balancing phases (305, $I_{Pi}$) for the respective electrode in a determination stage preceding said stimulation stage such that the difference (real $I_{Pi}$ - real $I_{Ni}$) between the circulating balancing current pulse (305, $I_{Pi}$) minus the respective stimulation current pulse phase (304, $I_{Ni}$) through the respective electrode satisfy:

- For at least one stimulating electrode, the difference equals an automatically determined positive value which may be different for different electrodes;
- For at least one returning electrode, the difference is positive and smaller or equal than the minimum among the difference values for the stimulating electrodes;
- For each electrode, both a DC blocking capacitor ($C_i$) which couples the respective electrode to a current source, a current sink, or a voltage, and a double layer capacitance ($C_{dli}$), wherein the double layer capacitance ($C_{dli}$) is formed by each electrode and the respective adjacent material, charge in the same direction and opposite for stimulating and returning electrodes, and wherein

- in said stimulation stage at least one of the electrodes is monitored, wherein, when a voltage ($\Delta V_{dli}$) accumulated at the double layer of the at least one monitored electrode crosses pre-defined thresholds ($-\Delta V_{AddOCP}$, $\Delta V_{SubOCP}$), correction currents ($I_{CORRStim}$, $I_{CORRRet}$) are generated and applied that reduce or cancel said accumulated voltages ($\Delta V_{dli}$).

FIG. 1

EP 3 216 490 A1

FIG. 2a

200

202

$V_{STIM}$

$V_{CounterP}$

205

$C_i$

204    206

203

$R_p$

$V_{CounterN}$

$V_{SS}$

## FIG. 2b

FIG. 3

**FIG. 4**

EP 3 216 490 A1

FIG. 5

FIG. 6

**FIG. 7**

Stimulation Phase

Balance Phase

FIG. 8

FIG. 9

FIG. 10

$V_I^*$

$V_i^*$

$V_N^*$

MUX
N:3

601

+
AMP
−

$V_{iBUF}^*$

## FIG. 11a

$V_1^*$

$V_{Stim}^*$

$V_{Ret}^*$

$V_{For}^*$

$V_N^*$

MUX
N:3

$V_{MUXStim}^*$

601

$V_{MUXRet}^*$

$V_{MUXFor}^*$

## FIG. 11b

FIG. 12

EP 3 216 490 A1

$\Delta V_{dli} \leq \Delta V_{SubOCP}$

$V_{REFStim}\text{-}P\Delta V_{CStim}$ | Per Pulse

$V^*_{MUXStim}$

801

**FIG. 13**

$\Delta V_{dli} \leq -\Delta V_{AddOCP}$

$V_{REFRet} \text{ -}P\Delta V_{CRet}$ | Per Pulse

$V^*_{MUXRet}$

901

$\Delta V_{dli} \leq -\Delta V_{AddOCP}$

$V_{REFRet} \text{ -}P\Delta V_{CFor}$ | Per Pulse

$V^*_{MUXFor}$

902

**FIG. 14**

**Correction Phase**

$I_{CORRStim}$  $C_W$  W

$I_{CORRStim}$  $C_X$  X

$C_Y$  Y

$I_{CORRRet}$

104

$C_Z$  Z

Forced to
$2I_{CORRStim} - I_{CORRRet}$

**FIG. 15**

**FIG. 16**

Output = Stim, Ret, For

**FIG. 17**

Europäisches
Patentamt

European
Patent Office

Office européen
des brevets

# EUROPEAN SEARCH REPORT

**Application Number**

EP 17 15 9839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/004423 A1 (BOVEJA BIRINDER R [US] ET AL) 5 January 2006 (2006-01-05) * abstract; claim *; figure * * | 1-16 | INV. A61N1/36 A61N1/362 |
| X,D | US 2014/257428 A1 (ZHU CHANGFANG [US]) 11 September 2014 (2014-09-11) * abstract; claim *; figure * * | 1 | |
| A | EP 2 508 225 A1 (GREATBATCH LTD [US]) 10 October 2012 (2012-10-10) * the whole document * | 1-16 | |
| A | EP 2 520 327 A2 (GREATBATCH LTD [US]) 7 November 2012 (2012-11-07) * the whole document * | 1-16 | |

**TECHNICAL FIELDS
SEARCHED      (IPC)**

A61N

The present search report has been drawn up for all claims

| Place of search | Date of completion of the search | Examiner |
|---|---|---|
| Munich | 25 July 2017 | Scheffler, Arnaud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 17 15 9839

This annex lists the patent family members relating to the patent documents cited in the above-mentioned European search report.
The members are as contained in the European Patent Office EDP file on
The European Patent Office is in no way liable for these particulars which are merely given for the purpose of information.

25-07-2017

| Patent document cited in search report | | | Publication date | Patent family member(s) | | | Publication date |
|---|---|---|---|---|---|---|---|
| US 2006004423 | A1 | | 05-01-2006 | NONE | | | |
| US 2014257428 | A1 | | 11-09-2014 | US | 2014257428 | A1 | 11-09-2014 |
| | | | | US | 2016106985 | A1 | 21-04-2016 |
| | | | | US | 2017143964 | A1 | 25-05-2017 |
| EP 2508225 | A1 | | 10-10-2012 | EP | 2508225 | A1 | 10-10-2012 |
| | | | | US | 2012259382 | A1 | 11-10-2012 |
| EP 2520327 | A2 | | 07-11-2012 | EP | 2520327 | A2 | 07-11-2012 |
| | | | | EP | 3053627 | A1 | 10-08-2016 |
| | | | | US | 2012277822 | A1 | 01-11-2012 |
| | | | | US | 2013310894 | A1 | 21-11-2013 |

EPO FORM P0459

For more details about this annex : see Official Journal of the European Patent Office, No. 12/82

**EP 3 216 490 A1**

### Patent documents cited in the description

- US 8977363 B2
- US 8165672 B2
- US 20140257428 A1